# EUROPEAN PATENT APPLICATION

(11) **EP 3 871 702 A1**
(43) Date of publication of application: **01.09.2021**
(21) Application number: 19875491.3
(22) Date of filing: 02.10.2019
(51) Int. Cl.: A61L 27/52, A61L 27/24

(54) **HYDROGEL FILM AND USE THEREOF**

(30) Priority: 26.10.2018 JP 2018201946
(71) Applicant: NATIONAL AGRICULTURE AND FOOD RESEARCH ORGANIZATION, Tsukuba-shi, Ibaraki 305-8517 (JP)
(72) Inventor: TAKEZAWA, Toshiaki, Tsukuba-shi, Ibaraki 305-8634 (JP)
(74) Representative: V.O.
(86) International application number: PCT/JP2019/038854
(87) International publication number: WO 2020/085018

(57) **Abstract**

A hydrogel membrane including a plurality of through-holes penetrating one surface and the other surface.

## Description

### [Technical Field]

The present invention relates to a hydrogel membrane and use thereof. More specifically, the present invention relates to a hydrogel membrane, a dried product of the hydrogel membrane,, an apparatus for producing the hydrogel membrane, or the dried product of the hydrogel membrane,, a method for producing a hydrogel, a method for producing a hydrogel membrane, a method for producing a dried product of a hydrogel, and a method for producing a dried product of a hydrogel membrane.

Priority is claimed on Japanese Patent Application No. 2018-201946, filed October 26, 2018, the content of which is incorporated herein by reference.

### [Background Art]

In the treatment of early-stage esophageal cancer or lesions diagnosed as Barrett's esophagus, mucosal resection of the esophagus may be performed. As a result, postoperative pathological tissue contraction due to the formation of inflammatory granulation tissue and excessive proliferation of myofibroblasts may occur, resulting in the development of esophageal stricture. On the other hand, Patent Literature 1 and Non-Patent Literature 1 disclose that the effect of preventing esophageal stricture and promoting epithelialization can be obtained by applying atelocollagen vitrigel membranes to mucosal resections of the esophagus. Similar treatments may be useful for gastrointestinal tracts other than the esophagus. Further, not only in the medical and pharmaceutical field, but there is also generally a demand for hydrogel membranes to adhere to uneven surfaces.

### [Citation List]

### [Patent Literature]

[Patent Literature 1]
PCT International Publication No. WO2017/110776

### [Non-Patent Literature]

[Non-Patent Literature 1]
Aoki S., et al., High-density collagen patch prevents stricture after endoscopic circumferential submucosal dissection of the esophagus: a porcine model, Gastrointest Endosc., 85 (5), 1076-1085, 2017

### [Summary of Invention]

### [Technical Problem]

However, a surgical clip is required to fix the collagen vitrigel membrane disclosed in Patent Literature 1 to the wound. Further, as described in Non-Patent Literature 1, it is very difficult to adhere the atelocollagen vitrigel membrane to a wide range of wounds. This is due to the unevenness of the wound in the esophageal lining, the exudate exuding from the wound, and the motility of the esophagus. Therefore, an object of the present invention is to provide a technique for adhering a hydrogel membrane to an uneven surface.

### [Solution to Problem]

The present invention includes the following aspects.
[1] A hydrogel membrane including a plurality of through-holes penetrating one surface and the other surface.
[2] The hydrogel membrane according to [1], wherein an opening area of the through-holes on the one surface is different from an opening area on the other surface.
[3] The hydrogel membrane according to [1] or [2], wherein the through-holes have a diameter of 0.1 µm to 5 mm.
[4] The hydrogel membrane according to [1] or [2], wherein the through-holes have a number density of 1 to 1,000,000/cm².
[5] The hydrogel membrane according to any one of [1] to [4], wherein the hydrogel contains native collagen or atelocollagen.
[6] A dried product of the hydrogel membrane according to any one of [1] to [5].
[7] An apparatus for producing the hydrogel membrane according to any one of [1] to [5] or the dried product of the hydrogel membrane according to [6], the apparatus including a container portion having a bottom surface portion and a side surface portion, and a plurality of columnar jigs configured to be arranged in contact with the bottom surface portion.
[8] A method for producing a hydrogel having a plurality of through-holes penetrating one surface and the other surface, the method including:
   (a) injecting a sol into a container portion of a production apparatus including the container portion having a bottom surface portion and a side surface portion and a plurality of columnar jigs configured to be arranged in contact with the bottom surface portion;
   (b) arranging the plurality of columnar jigs to be in contact with the bottom surface portion before or after injecting the sol;
   (c) allowing the sol to stand and gelate to obtain a hydrogel; and
   (d) removing the plurality of columnar jigs to obtain a hydrogel having a plurality of through-holes penetrating one surface and the other surface.
[9] A method for producing a dried product of a hydrogel having a plurality of through-holes penetrating one surface and the other surface, the method including:
   (e) drying the hydrogel obtained by the production method according to [8] to obtain a dried product of the hydrogel.
[10] A method for producing a dried product of a hydrogel membrane having a plurality of through-holes penetrating one surface and the other surface, the method including:
   (f) hydrating the dried product of the hydrogel obtained by the production method according to [9] to obtain a hydrated hydrogel membrane.
[11] A method for producing a dried product of a hydrogel membrane having a plurality of through-holes penetrating one surface and the other surface, the method including:
   (e2) drying the hydrogel membrane obtained by the production method according to [10] to obtain a dried product of the hydrogel membrane.

### [Advantageous Effects of Invention]

According to the present invention, it is possible to provide a technique for adhering a hydrogel membrane to an uneven surface.

### [Brief Description of Drawings]

Fig. 1A is a schematic view illustrating an example of a hydrogel membrane of the present embodiment. Fig. 1B is a cross-sectional view taken along a line b-b' of Fig. 1A.
Fig. 2 is a photograph showing an example of a production apparatus of the present embodiment.
Fig. 3A is a photograph showing a state in which a side surface portion of the production apparatus is removed from a hydrogel membrane in Production Example 1. Fig. 3B is a photograph showing a state in which a columnar member is removed from the hydrogel membrane in Production Example 1. Figs. 3C and 3d are photographs of the hydrogel taken out from the production apparatus in Production Example 1.
Figs. 4A and 4B are photographs of a hydrogel dried product after vitrification in Production Example 1. Fig. 4C is a photograph of the hydrogel membrane after a first rehydration in Production Example 1. Fig. 4D is a photograph of the hydrogel membrane after a second rehydration in Production Example 1. Fig. 4E is a photograph of the hydrogel membrane after a third rehydration in Production Example 1.
Figs. 5A to 5D and 5F are photographs taken in steps in Production Example 6. Fig. 5E is a photomicrograph of a through-hole of the hydrogel dried product after vitrification, and Fig. 5G is a photomicrograph of the through-hole of the hydrogel membrane after rehydration.
Figs. 6A, 6D, 6G, 6J, and 6M are photographs of hydrogel membranes of Production Examples 1 to 5, respectively. In addition, Figs. 6B and 6C, 6E and 6F, 6H and 6I, 6K and 6L, and 6N and 6O are photomicrographs of the hydrogel membranes of Production Examples 1 to 5, respectively.
Fig. 7A is a photograph showing the result of adhering an atelocollagen vitrigel membrane having no through-holes to an uneven surface in Experimental Example 2. Fig. 7B is a photograph showing the result of adhering the hydrogel membrane of Production Example 2 to the uneven surface in Experimental Example 2. Fig. 7C is a photograph showing the result of adhering the hydrogel membrane of Production Example 1 to the uneven surface in Experimental Example 2.
Fig. 8 is a photograph showing a state in which each hydrogel membrane is covered on a conical tube in Experimental Example 3.
Fig. 9A is a photograph showing a state in which a conical tube to which an atelocollagen vitrigel membrane having no through-holes is fixed is inverted in Experimental Example 3. Fig. 9B is a photograph showing a state in which the inverted conical tube is brought into contact with cardboard in Experimental Example 3. Fig. 9C is a photograph of Experimental Example 3 in which no trace of water was observed as a result of contact with the cardboard.
Fig. 10 is a photograph showing a state in which a conical tube to which the hydrogel membrane of Production Example 2 is fixed is inverted in Experimental Example 3.
Fig. 11A is a photograph showing a state in which a conical tube to which a hydrogel membrane of Production Example 1 is fixed is inverted in Experimental Example 3. Fig. 11B is a photograph showing a state in which the inverted conical tube is brought into contact with the cardboard about 15 minutes after the conical tube is inverted in Experimental Example 3. Fig. 11C is a photograph of Experimental Example 3 in which traces of water are observed as a result of contacting with the cardboard.

### [Description of Embodiments]

### [Hydrogel membrane]

In one embodiment, the present invention provides a hydrogel membrane having a plurality of through-holes penetrating one surface and the other surface.

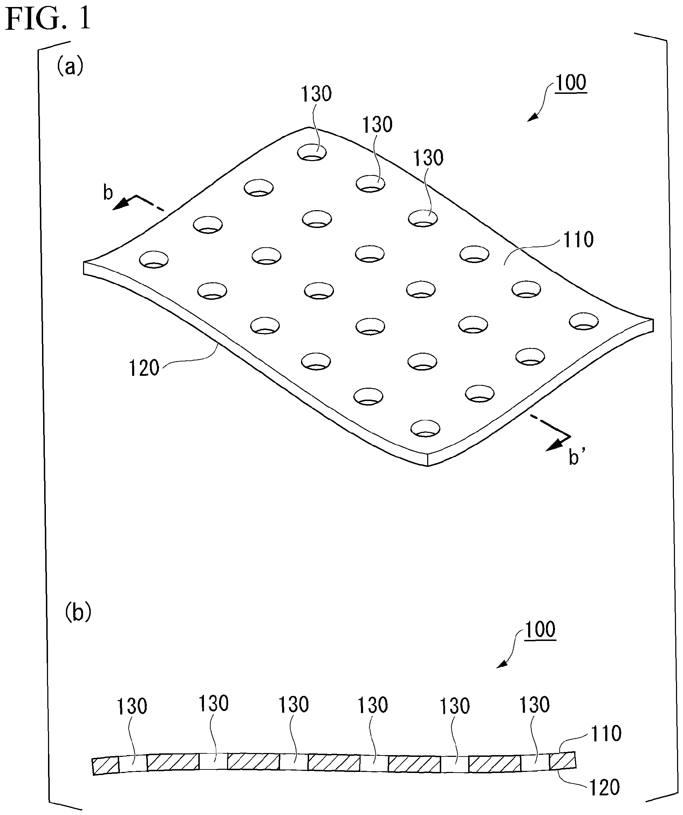

Figs. 1 is schematic views illustrating an example of a hydrogel membrane of the present embodiment. Fig. 1A is a perspective view of a hydrogel membrane 100. The hydrogel membrane 100 has one surface 110 and the other surface 120. Further, the hydrogel membrane 100 has a plurality of through-holes 130 penetrating the one surface 110 and the other surface 120. Fig. 1B is a cross-sectional view taken along a line b-b' of Fig. 1A.

As will be described later in the examples, the hydrogel membrane of the present embodiment can be easily adhered to an uneven surface. Further, even in a case where the uneven surface is the wound of a living body and exudate exudes from the uneven surface, the exudate can pass through the through-hole, so that the adhesion of the hydrogel membrane to an uneven surface can be maintained. As used herein, the "hydrogel membrane having through-holes" may be referred to as a "porous hydrogel membrane".

As used herein, the term "sol" means one in which dispersoid colloidal particles (particle size of about 1 to several hundred nm) using a liquid as a dispersion medium are particularly composed of a polymer compound. More specific examples of the sol include an aqueous solution of a natural polymer compound or a synthetic polymer compound. Therefore, in a case where these polymer compounds form a network structure by chemical bonding, they are transferred to "hydrogel", which is a semi-solid substance having a large amount of water in the network. That is, "hydrogel" means one obtained by gelating a sol. More specific examples of the hydrogel include those obtained by introducing crosslinks into artificial materials such as natural polymer compounds and synthetic polymer compounds to gelate them.

Further, as used herein, the "membrane" can also be referred to as a sheet. The thickness of the hydrogel membrane of the present embodiment may be appropriately set as necessary, and may be, for example, 0.1 µm to 5 mm, for example, 2 µm to 1 mm, for example, 20 µm to 400 µm. As used herein, a hydrogel having a thickness of more than 5 mm is referred to as a hydrogel, and may be distinguished from a hydrogel membrane.

Further, the opening area of the through-holes on the one surface of the hydrogel membrane may be the same as or different from the opening area of the through-holes on the other surface of the hydrogel membrane.

For example, the opening area of the through-holes on the one surface of the hydrogel membrane may be smaller than the opening area of the through-holes on the other surface of the hydrogel membrane. In this case, the area of the one surface of the hydrogel membrane is larger than the area of the other surface of the hydrogel membrane. In a case where such a hydrogel membrane is adhered to the uneven surface, when the one surface of the hydrogel membrane is adhered so as to face the uneven surface, the hydrogel membrane tends to be adhered more favorably than when the other surface is adhered so as to face the uneven surface.

Further, for example, in a case where the uneven surface is a wound of a living body, since the opening area of the through-holes is small but open on the one surface of the hydrogel membrane, the exudate can be passed through the through-holes and the adhesion of the hydrogel membrane to an uneven surface can be maintained.

In a case where the opening area of the through-holes on the one surface of the hydrogel membrane is different from the opening area of the through-holes on the other surface of the hydrogel membrane, the ratio of the opening area of the through-holes on the one surface of the hydrogel membrane to the opening area of the through-holes on the other surface of the hydrogel membrane may be, for example, about 100:1 to 1:100, 50:1 to 1:50, or 10:1 to 1:10.

The hydrogel membrane in which the opening area of the through-holes on one surface of the hydrogel membrane and the opening area of the through-holes on the other surface of the hydrogel membrane are the same can be produced by, for example, forming the through-holes by hollowing out the hydrogel membrane by using a cylindrical blade or the like after forming the hydrogel membrane. Examples of the cylindrical blade include a medical device such as a trepan. The shape of the blade may be appropriately changed according to the cross-sectional shape of the through-hole, and is not limited to the cylindrical shape.

Alternatively, the hydrogel membrane in which the opening area of the through-holes on one surface of the hydrogel membrane and the opening area on the other surface of the hydrogel membrane are same can be produced by forming the through-holes in which the opening area on the one surface and the opening area on the other surface are the same by laser irradiation after forming the hydrogel membrane.

The hydrogel membrane in which the opening area of the through-holes on one surface of the hydrogel membrane and the opening area of the through-holes on the other surface of the hydrogel membrane are different can be produced by, for example, by laser irradiation after forming the hydrogel membrane. Here, by adjusting the laser irradiation angle and the like, it is possible to produce a hydrogel membrane in which the opening area of the through-holes on one surface of the hydrogel membrane and the opening area of the through-holes on the other surface of the hydrogel membrane are different.

Alternatively, the hydrogel membrane in which the opening area of the through-holes on one surface of the hydrogel membrane and the opening area of the through-holes on the other surface of the hydrogel membrane are different can be produced by a production method described later using a production apparatus described later.

However, in the method of hollowing out the hydrogel membrane with a blade to form through-holes, the material of the hollowed out portion is wasted. In addition, when through-holes are formed in the hydrogel membrane by laser irradiation, the material of the hollowed out portion by the laser irradiation is wasted, and the portion irradiated with the laser may be denatured by heat.

The area of the hydrogel membrane of the present embodiment may be appropriately set as necessary, and may be, for example, 1 mm² to 400 cm², for example, 20 mm² to 40 cm², for example, 80 mm² to 4 cm².

As described above, the hydrogel membrane of the present embodiment has through-holes. The shape of the plane perpendicular to the axial direction of the through-holes (hereinafter, may be referred to as the cross-sectional shape of the through-holes) is not particularly limited, and examples thereof include polygons such as a triangle, a quadrangle (including a square, a rectangle, and a trapezoid), a pentagon, a hexagon, a heptagon, and an octagon; and a circle, an ellipse, an approximate circle, an ellipse, an approximate ellipse, a semicircle, and a fan. In one through-hole, the cross-sectional shape of the through-hole may be constant or may change in the middle.

Further, the hydrogel membrane may have a plurality of kinds of through-holes having different shapes.

As used herein, the diameter of the through-holes of the hydrogel membrane refers to the diameter of a circle having the same area as the widest cross-sectional area among the cross-sectional areas of the plane perpendicular to the axial direction of the through-hole. The diameter of the through-hole of the hydrogel membrane of the present embodiment may be 0.1 µm to 5 mm, 1 µm to 2 mm, or 10 µm to 1 mm.

The diameter of the through-hole may not be constant. For example, a plurality of kinds of through-holes having different diameters may be mixed. Alternatively, the diameter of the through-holes may differ depending on the position of the hydrogel membrane.

In the hydrogel membrane of the present embodiment, the number density of the through-holes may be set as necessary, and may be 1 to 1,000,000/cm², 1 to 10,000/cm², or may be 1 to 100/cm². In a case where the diameter of the through-holes is about 0.1 µm, the number density of the through-holes may be more than 1,000,000/cm².

Further, the number density of the through-holes may differ depending on the position of the hydrogel membrane. For example, the central portion of the hydrogel membrane may have a higher number density of through-holes than the surroundings. Alternatively, the number density of through-holes in the central portion of the hydrogel membrane may be lower than that in the periphery.

Examples of the sol that is the material of the hydrogel membrane of the present embodiment include natural polymer compounds such as extracellular matrix-derived components that gelate, fibrin, agar, agarose, and cellulose, and synthetic polymer compounds such as polyacrylamide, polyvinyl alcohol, polyethylene oxide, and poly(II-hydroxyethylmethacrylate)/polycaprolactone.

Examples of the above-mentioned extracellular matrix-derived component that gelates include, but are not limited to, collagen (type I, type II, type III, type V, type XI, etc.), basement membrane components reconstituted from mouse EHS tumor extracts (contains type IV collagen, laminin, heparan sulfate proteoglycans, etc.) (trade name "Matrigel"), glycosaminoglycans, hyaluronic acid, proteoglycans, and gelatin. A desired hydrogel membrane can be produced by selecting components such as salts most suitable for each gelation, concentrations, and pH thereof. Among them, the sol is preferably an extracellular matrix-derived component that gelates, and more preferably collagen. Further, among collagens, still more preferred is native collagen or atelocollagen.

### [Dried product of hydrogel membrane]

In one embodiment, the present invention provides a dried product of the hydrogel membrane described above. The hydrogel membrane described above may be dried to form a dried product. By drying the hydrogel membrane, the free water in the hydrogel can be completely removed, and further the partial removal of the bound water can be advanced. By rehydrating the dried product of the present embodiment with phosphate-buffered saline (PBS), a culture medium, or the like, vitrigel (registered trademark) can be obtained.

"Vitrigel" refers to a gel in a stable state obtained by rehydration of a conventional hydrogel that has been vitrified by drying (vitrification), and is named by the inventor, "vitrigel". (Registered trademark)". In the following, when the term "vitrigel" is used, the term "(registered trademark)" may be omitted.

The longer the period of the vitrification step, that is, the step of completely removing the free water in the hydrogel and then proceeding with the partial removal of the bound water, the more the vitrigel having excellent transparency and strength can be obtained in the case of being rehydrated. As necessary, the vitrigel obtained by rehydration after drying for a short period of time may be washed with PBS or the like and vitrified again.

As a drying method, for example, various methods such as air drying, drying in a closed container (circulating the air in a container and constantly supplying dry air), and drying in an environment in which silica gel is arranged may be used. For example, the air drying method includes drying in a thermo-hygrostat kept sterile at 10°C and 40% relative humidity for about two days, and drying in a clean bench with a sterile condition for about 24 hours at room temperature.

Further, as used herein, the dried product of hydrogel that has not been rehydrated after drying may be simply referred to as "hydrogel dried product". Then, the gel obtained by hydrating the hydrogel after drying may be distinguished and represented as "vitrigel", and the dried product obtained by drying the vitrigel may be referred to as "vitrigel dried product". Furthermore, as used herein, the hydrogel in the form of a membrane may be simply referred to as a "hydrogel membrane", and the vitrigel in the form of a membrane may be simply referred to as a "vitrigel membrane".

Further, the dried product of the obtained vitrigel membrane may be irradiated with ultraviolet rays. A known ultraviolet irradiation device can be used for the irradiation of ultraviolet rays. In the irradiation energy of ultraviolet rays to the vitrigel membrane dried product, the total irradiation dose per unit area is preferably 0.1 mJ/cm² to 6000 mJ/cm², more preferably 10 mJ/cm² to 4000 mJ/cm², and still more preferably 100 mJ/cm² to 3000 mJ/cm². When the total irradiation dose is in the above range, the transparency and strength of the vitrigel membrane material obtained by the subsequent rehydration can be made particularly preferable.

Further, the irradiation of the vitrigel membrane dried product with ultraviolet rays may be repeated a plurality of times. In the case of repeating the irradiation of the vitrigel membrane dried product with ultraviolet rays, it is preferable to perform the step of rehydration and revitrification of the vitrigel membrane dried product that has been subjected to the ultraviolet irradiation treatment after the first irradiation of ultraviolet rays, and then perform irradiation of ultraviolet rays to the dried product of the vitrigel membrane material after the second and subsequent revitrification.

When the total irradiation dose of ultraviolet rays per unit area is the same, the vitrigel membrane material obtained in the subsequent rehydration step can be further enhanced in transparency and strength by repeatedly irradiating the vitrigel membrane dried product with ultraviolet rays by dividing it into a plurality of times. Further, the larger the number of divisions, the more preferable. For example, when the total irradiation dose per unit area of irradiation of ultraviolet rays to the vitrigel membrane dried product is in the range of 1,000 mJ/cm² to 4,000 mJ/cm², the number times of irradiation in the range may be 2 to 10 times, or 2 to 6 times.

Further, in a case where the irradiation of the vitrigel membrane dried product with ultraviolet rays is repeated, the irradiation site of the ultraviolet rays may be irradiated separately on one surface and the other surface of the vitrigel membrane dried product, and the total irradiation dose thereof may be set as the total irradiation dose of ultraviolet rays per unit area to the vitrigel membrane dried product.

The reason why the strength and transparency of the vitrigel membrane material obtained in the subsequent rehydration step are enhanced by irradiating the vitrigel membrane dried product with ultraviolet rays is considered that the polymer compounds in the vitrigel membrane material are crosslinked by the ultraviolet rays. That is, the operation can impart high transparency and strength to the vitrigel membrane material.

Furthermore, the obtained vitrigel membrane dried product that has been subjected to the ultraviolet irradiation treatment may be rehydrated with PBS, a culture medium, or the like to obtain the vitrigel membrane material. Furthermore, the obtained vitrigel membrane material may be dried to be revitrified to obtain a dried product of the vitrigel membrane material.

As used herein, the product obtained by irradiating a vitrigel membrane dried product with ultraviolet rays may be referred to as "vitrigel membrane material obtained by subjecting a vitrigel membrane dried product to an ultraviolet irradiation treatment", the gel membrane obtained by hydrating the vitrigel membrane material may be referred to as "vitrigel membrane material", and the dried product obtained by drying the vitrigel membrane material may be referred to as "dried product of vitrigel membrane material". Therefore, the "vitrigel membrane" and the "vitrigel membrane material" are hydrates.

### [Production apparatus]

In one embodiment, the present invention provides a production apparatus including a container portion having a bottom surface portion and a side surface portion, and a plurality of columnar jigs configured to be arranged in contact with the bottom surface portion. The production apparatus of the present embodiment produces the hydrogel membrane or the dried product of the hydrogel membrane described above.

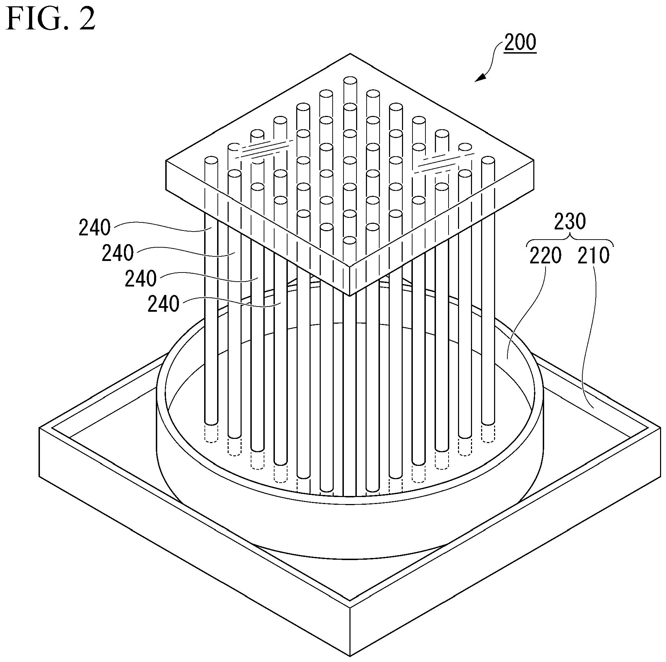

Fig. 2 is a photograph showing an example of a production apparatus of the present embodiment. As shown in Fig. 2, the production apparatus 200 includes a container portion 230 having a bottom surface portion 210 and a side surface portion 220, and a plurality of columnar jigs 240 configured to be arranged in contact with the bottom surface portion 210.

As will be described later, in the production of the hydrogel membrane, first, a sol is injected into the container portion 230. Subsequently, the sol is gelated in a state where the columnar jigs 240 are arranged so as to be in contact with the bottom surface portion 210. After that, when the columnar jigs 240 are removed, a hydrogel in which the portion where the columnar jigs 240 were present remains as through-holes is obtained. That is, the through-holes of the hydrogel are formed by the columnar jigs 240. Since the gelation occurs in a state in which the columnar jigs 240 are in contact with the bottom surface portion 210, the through-holes formed are those that penetrate one surface and the other surface of the hydrogel.

Further, the through-holes on the bottom surface side of the hydrogel tend to be pushed from the surroundings by the weight of the hydrogel membrane and become narrower. Therefore, the opening area of the through-holes on the bottom surface side surface of the hydrogel tends to be smaller than the opening area of the through-holes on the other surface of the hydrogel membrane.

Examples of the materials of the production apparatus 200 constituted by the bottom surface portion 210, the side surface portions 220, and the columnar jigs include, but are not limited to, glass material, polyacrylate (acrylic resin), polystyrene, nylon, and stainless steel.

More specific examples of the glass material include soda-lime glass, Pyrex (registered trademark) glass, Vycor (registered trademark) glass, and quartz glass. More specifically, example of the polyacrylates (acrylic resin) include, for example, poly(methyl methacrylate), poly(ethyl methacrylate), poly(butyl methacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

It is also preferable that at least the side surface portion 220 is transparent, as it makes it easier to inject the sol into the container portion 230 and check the gelation during hydrogel production.

### [Production method]

### (First embodiment)

In one embodiment, the present invention provides a method for producing a hydrogel having a plurality of through-holes penetrating one surface and the other surface, which includes (a) injecting a sol into a container portion 230 of the production apparatus described above, (b) arranging the plurality of columnar jigs 240 to be in contact with the bottom surface portion 210 before or after injecting the sol, (c) allowing the sol to stand and gelate to obtain a hydrogel, and (d) removing the plurality of columnar jigs 240 to obtain a hydrogel having a plurality of through-holes penetrating one surface and the other surface.

The produced hydrogel having a plurality of through-holes may be a vitrigel membrane having a plurality of through-holes by repeating the drying step and the hydration step once or a plurality of times. That is, the production method of the present embodiment may be a production method of a hydrogel membrane which further includes (e) drying the hydrogel obtained in (d) to obtain a dried product, and (f) hydrating the dried product to obtain a hydrated hydrogel membrane.

In a case where the sol injected in (a) is a collagen sol, the collagen sol may be prepared by using, for example, physiological saline, phosphate-buffered saline (PBS), HBSS (Hank's Balanced Salt Solution), a basic culture medium, a serum-free culture medium, a serum-containing culture medium, or the like, as a sol having an optimum salt concentration. Further, the pH of the solution at the time of gelation of collagen may be, for example, 6 to 8. Further, the collagen sol may be prepared at, for example, about 4°C.

In particular, in a case where a serum-free culture medium is used, it is possible to prevent the hydrogel from containing substances (for example, antigens, virulence factors, etc.) that are contained in the other serum components of animals and are not suitable for application to the wound of a living body. Therefore, the hydrogel membrane obtained by using the serum-free culture medium may be suitably used for medical purposes.

The concentration of the collagen sol for producing the hydrogel is preferably 0.1 to 1.0% by mass, more preferably 0.2 to 0.6% by mass. When the collagen sol concentration is the lower limit value or more, gelation is not too weak, and when the collagen sol concentration is the upper limit value or less, a hydrogel made of a uniform collagen gel can be obtained.

Subsequently, in (b), the plurality of columnar jigs 240 are arranged so as to be in contact with the bottom surface portion 210. This may be done before the injection of the sol into the container portion 230, or after the injection.

Subsequently, in (c), the sol is allowed to stand and gelate to obtain a hydrogel. The temperature at which the sol is kept warm can be appropriately adjusted according to the kind of sol used. For example, in a case the sol is a collagen sol, the heat retention at the time of gelation can be set to a temperature lower than the denaturation temperature of the collagen depending on the animal species of the collagen to be used, and in general, gelation can be performed in several minutes to several hours by keeping warm at a temperature 20°C or more and 37°C or less.

Subsequently, in (d), the plurality of columnar jigs 240 are removed to obtain a hydrogel having a plurality of through-holes penetrating one surface and the other surface. The hydrogel may be used as it is, or may be used as a vitrigel membrane by repeating drying and hydration in (e) and (f) to produce a vitrigel membrane.

### (Second embodiment)

In one embodiment, the present invention provides a method for producing a dried product of a hydrogel having a plurality of through-holes penetrating one surface and the other surface, which includes (a) injecting a sol into a container portion 230 of the production apparatus described above, (b) arranging the plurality of columnar jigs 240 to be in contact with the bottom surface portion 210 before or after injecting the sol, (c) allowing the sol to stand and gelate to obtain a hydrogel, (d) removing the plurality of columnar jigs 240 to obtain a hydrogel having a plurality of through-holes penetrating one surface and the other surface, and (e) drying the hydrogel obtained in (d) to obtain a dried product of the hydrogel.

The produced dried hydrogel may be obtained as a vitrigel membrane dried product by repeating the hydration step and the drying step once or a plurality of times. That is, the production method of the present embodiment may be a production method which further includes (f) hydrating the dried product of the hydrogel obtained in (e) to obtain a hydrated hydrogel membrane, and (e2) drying the hydrogel membrane obtained in (f) to obtain a dried product of the hydrogel membrane.

In the production method of the present embodiment, (a) to (e) are the same as those described above. Further, (e2) is the same as (e) except that the object to be dried is a hydrogel or a hydrogel membrane.

### (Third embodiment)

The hydrogel can be produced as follows without using the apparatus described above. First, a sol is injected into a suitable container. Subsequently, the sol is allowed to stand and gelate to obtain a hydrogel. Subsequently, the hydrogel is hollowed out using a cylindrical blade or the like to form through-holes. Examples of the cylindrical blade include a medical device such as a trepan. The shape of the blade may be appropriately changed according to the cross-sectional shape of the through-hole, and is not limited to the cylindrical shape.

Alternatively, through-holes may be formed in the hydrogel membrane by laser irradiation after forming the hydrogel membrane.

The produced hydrogel (membrane) may be dried to obtain a dried product of the hydrogel (membrane). Alternatively, the drying step and the hydration step may be repeated once or a plurality of times to obtain a vitrigel membrane or a dried product of a vitrigel membrane.

### [Examples]

Next, the present invention will be described in more detail with reference to Examples, but the present invention is not limited to the following Examples.

### [Production Example 1]

A hydrogel membrane was produced using the production apparatus shown in Fig. 2. As the columnar member, a cylindrical stainless steel shaft having a diameter of 2 mm was used. The production apparatus was sterilized with 70% ethanol and dried in a clean bench.

Into a 50-mL conical tube on ice, 16 mL of serum-free culture medium was dispensed. Subsequently, 16 mL of porcine atelocollagen (manufactured by Kanto Chemical Co., Inc., collagen concentration 1% by mass) was added to prepare a uniform collagen sol. As the serum-free culture medium, DMEM medium (catalog number "11885-084", manufactured by Thermo Fisher Scientific) containing 20 mM HEPES (catalog number "15630-080", manufactured by Thermo Fisher Scientific), 100 units/mL penicillin, and 100 µg/mL streptomycin (catalog number "15140-148", manufactured by Thermo Fisher Scientific) was used.

Subsequently, 26.7 mL of sol was injected into the container portion of the production apparatus. Subsequently, the production apparatus was allowed to stand in an incubator set at 37°C and 5% CO₂ for 2 hours for gelation to obtain a hydrogel.

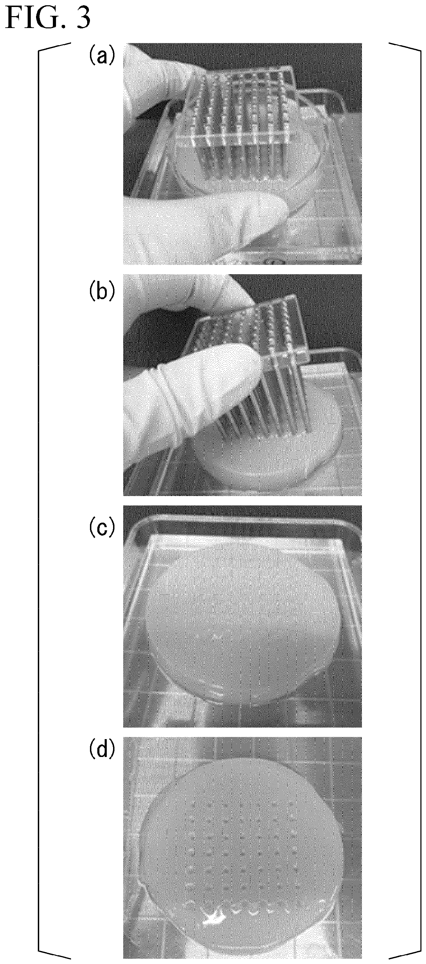

Subsequently, the free water in the hydrogel was dehydrated from the gap between the bottom surface portion and the side surface portion of the container and dried from the top surface, and at a time point at which the height of the hydrogel reached 6 mm, the porous hydrogel was taken out from the production apparatus, and dried and vitrified in a thermo-hygrostat kept sterile at 10°C and 40% relative humidity. Fig. 3A is a photograph showing a state in which a side surface portion of the production apparatus is removed from the hydrogel. Fig. 3B is a photograph showing a state in which a columnar member is removed from the hydrogel. Figs. 3C and 3d are photographs of the porous hydrogel taken out from the production apparatus.

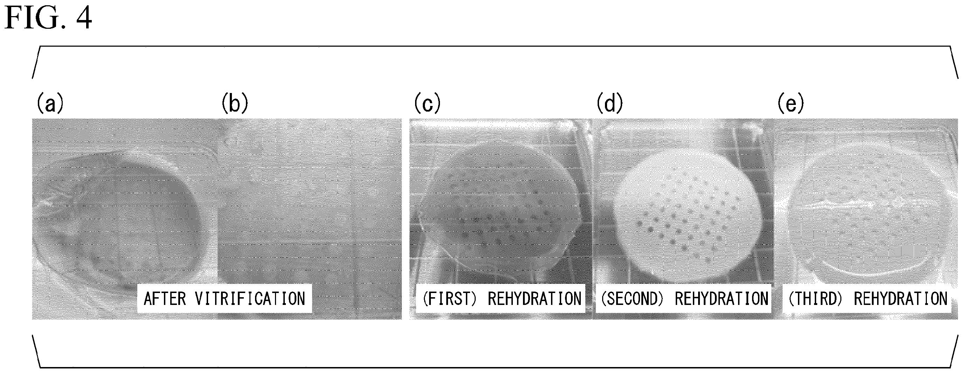

Subsequently, after vitrification, it was taken out from a thermo-hygrostat and rehydrated in PBS. Further, the washing step by rehydration was repeated with fresh PBS, and washing was performed a total of 3 times to obtain a hydrogel membrane of Production Example 1. Figs. 4A and 4B are photographs of a hydrogel dried product after vitrification. Fig. 4C is a photograph of the hydrogel membrane rehydrated by a first wash with PBS addition. Fig. 4D is a photograph of the hydrogel membrane rehydrated by a second wash with PBS addition. Fig. 4E is a photograph of a hydrogel membrane rehydrated by a third wash with PBS addition.

### [Production Example 2]

A hydrogel membrane having through-holes was produced using a mold having a bottom surface portion and a cylindrical side surface portion having an inner diameter of 34 mm. The through-holes were formed using a trepan. First, the mold was sterilized with 70% ethanol and dried in a clean bench. Subsequently, 10 mL of the same collagen sol as in Production Example 1 was injected into the mold. Subsequently, after standing for 30 minutes, the mold was allowed to stand in an incubator set at 37°C and 5% CO₂ for 2 hours for gelation to obtain a hydrogel.

Subsequently, the free water in the hydrogel was dehydrated from the gap between the bottom surface portion and the side surface portion of the mold and dried from the top surface, and at a time point at which the height of the hydrogel reached 6 mm, the hydrogel was taken out from the mold, and dried and vitrified in a thermo-hygrostat kept sterile at 10°C and 40% relative humidity.

Subsequently, after vitrification, it was taken out from a thermo-hygrostat and rehydrated in PBS. Further, the washing step by rehydration was repeated with fresh PBS, and after washing a total of 3 times, a trepan having a diameter of 2.5 mm was pressed to form through-holes, whereby a porous hydrogel membrane of Production Example 2 was formed.

### [Production Example 3]

A porous hydrogel membrane of Production Example 3 was obtained in the same manner as in Production Example 2 except that a trepan having a diameter of 1 mm was used.

### [Production Example 4]

A porous hydrogel membrane of Production Example 4 was obtained in the same manner as in Production Example 2 except that an 18G injection needle (diameter 1.2 mm) was used.

### [Production Example 5]

A porous hydrogel membrane of Production Example 5 was obtained in the same manner as in Production Example 2 except that a 27G injection needle (diameter 0.4 mm) was used.

### [Production Example 6]

A hydrogel membrane having through-holes was produced using a mold having a bottom surface portion and a cylindrical side surface portion having an inner diameter of 34 mm. The through-holes were formed using a trepan. First, the mold was sterilized with 70% ethanol and dried in a clean bench. Subsequently, 10 mL of the same collagen sol as in Production Example 1 was injected into the mold. Subsequently, after standing for 30 minutes, the mold was allowed to stand in an incubator set at 37°C and 5% CO₂ for 2 hours for gelation to obtain a hydrogel.

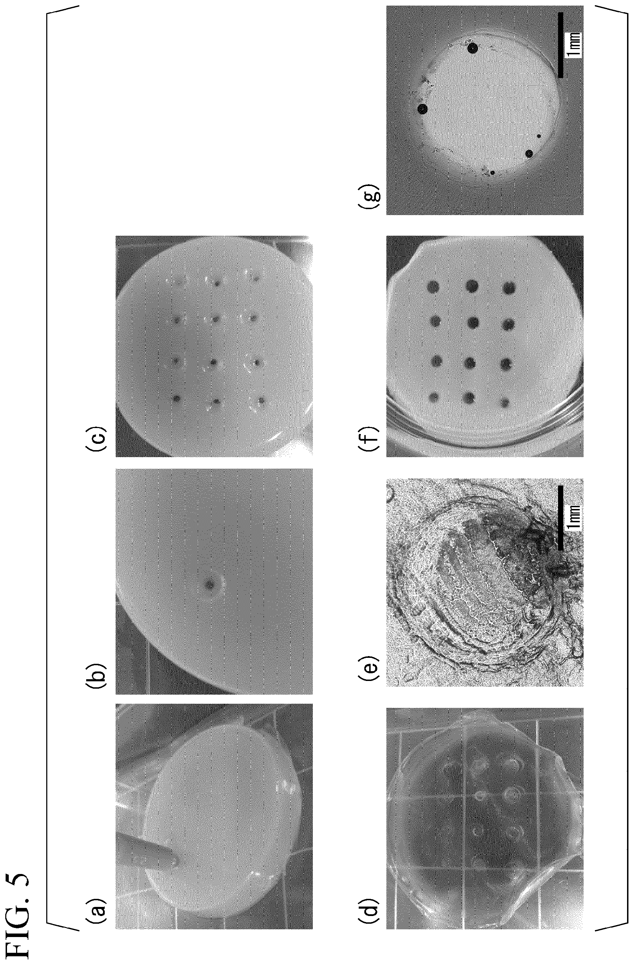

Subsequently, the free water in the hydrogel was dehydrated from the gap between the bottom surface portion and the side surface portion of the mold and dried from the top surface, and at a time point at which the height of the hydrogel reached 6 mm, the hydrogel was taken out from the mold. Subsequently, a trepan having a diameter of 2.5 mm was pressed to form the through-holes. Figs. 5A to 5C are photographs of the formation of the through-holes in the hydrogel using a trepan.

Subsequently, drying and vitrification were performed in a thermo-hygrostat kept sterile at 10°C and 40% relative humidity. Fig. 5D is a photograph of the vitrified hydrogel dried product. Further, a through-hole of the vitrified hydrogel dried product was observed with a microscope. Fig. 5E is a photomicrograph of the through-hole, and the scale bar is 1 mm.

Subsequently, the vitrified hydrogel dried product was removed from the thermo-hygrostat and rehydrated in PBS. Fig. 5F is a photograph of the rehydrated hydrogel membrane. Further, a through-hole of the rehydrated hydrogel membrane was observed with a microscope. Fig. 5G is a photomicrograph of the through-hole, and the scale bar is 1 mm.

Further, the washing step by rehydration was repeated with fresh PBS, and washing was performed a total of 3 times to obtain a porous hydrogel membrane of Production Example 6. The hydrogel membrane of Production Example 6 was mainly different from the hydrogel membrane of Production Example 2 in that through-holes were formed before the hydrogel was vitrified.

### [Production Example 7]

The porous hydrogel membrane of Production Example 7 was obtained in the same manner as in Production example 6, except that at the time point at which the height of the hydrogel reached 10 mm, the hydrogel was not taken out from the mold and a trepan having a diameter of 2.5 mm was pressed to form the through-holes. Since the blade of the trepan was 7 mm short of the height of the gel of 10 mm, the trepan was pressed against the hydrogel and then hollowed out with tweezers to form the through-holes.

### [Experimental Example 1]

### (Observation of hydrogel membrane)

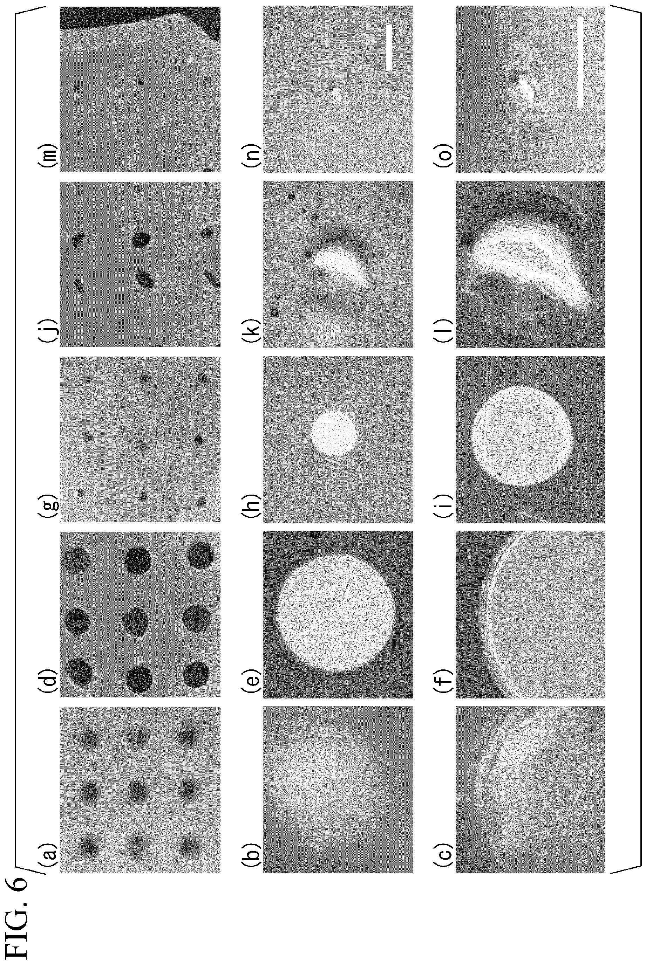

The hydrogel membranes of Production Examples 1 to 5 were observed. Figs. 6A, 6D, 6G, 6J, and 6M are photographs of hydrogel membranes of Production Examples 1 to 5, respectively. In addition, Figs. 6B and 6C, 6E and 6F, 6H and 6I, 6K and 6L, and 6N and 6O are photomicrographs of the hydrogel membranes of Production Examples 1 to 5, respectively. The magnifications of Figs. 6B, 6E, 6H, 6K, and 6N are the same, and the scale bar is 1 mm. Further, the magnifications of Figs. 6C, 6F, 6I, 6L, and 6O are the same, and the scale bar is 1 mm.

The through-holes of the hydrogel of Production Example 1 look unclear because the opening area of the through-holes on one surface of the hydrogel membrane is smaller than the opening area of the through-holes on the other surface of the hydrogel membrane.

### [Experimental Example 2]

### (Examination of adhesion to uneven surface)

The hydrogel membranes of Production Example 1 and Production Example 2 were adhered to an uneven surface, and the state was observed. Further, for comparison, the same examination was conducted on the atelocollagen vitrigel membrane having no through-holes.

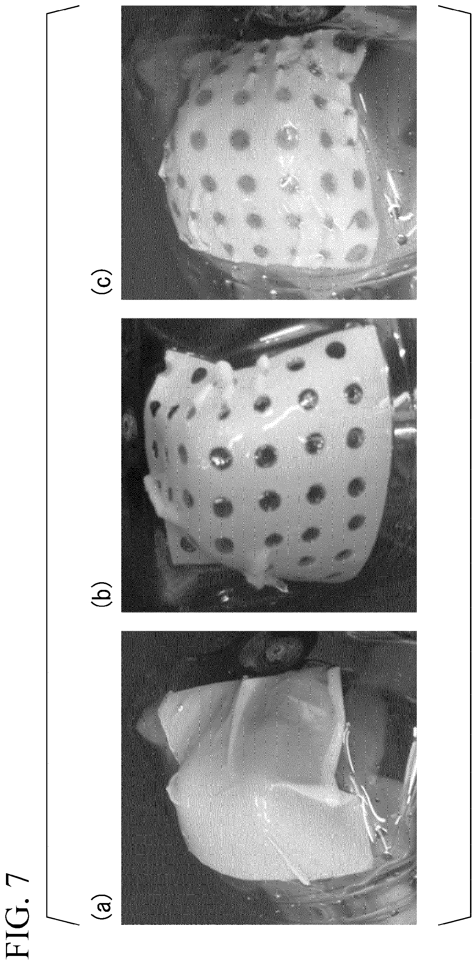

Fig. 7A is a photograph showing the result of adhering an atelocollagen vitrigel membrane having no through-holes to an uneven surface. Fig. 7B is a photograph showing the result of adhering the hydrogel membrane of Production Example 2 to an uneven surface. Fig. 7C is a photograph showing the result of adhering the hydrogel membrane of Production Example 1 to an uneven surface.

As a result, it was confirmed that the hydrogel membrane having through-holes has better adhesion to an uneven surface as compared with the hydrogel membrane having no through-holes.

### [Experimental Example 3]

### (Examination of liquid permeability)

The liquid permeability of the hydrogel membranes of Production Example 1 and Production Example 2 was examined. Further, for comparison, the same examination was conducted on the atelocollagen vitrigel membrane having no through-holes.

First, the mouths of 50-mL conical tubes containing 15 mL of water were covered with an atelocollagen vitrigel membrane having no through-holes and hydrogel membranes of Production Example 1 and Production Example 2, and dried.

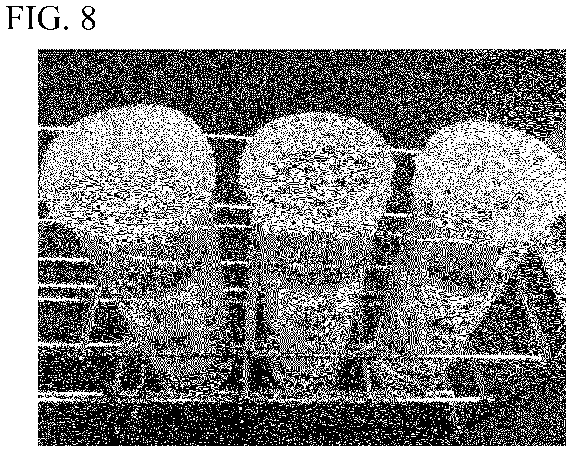

Subsequently, after the hydrogel membrane was dried, a parafilm was wrapped around the mouth of the conical tube to fix the dried product of each hydrogel membrane. Subsequently, the conical tube was inverted to observe how water permeated the hydrogel membrane. Fig. 8 is a photograph showing a state in which each hydrogel membrane was covered on a conical tube.

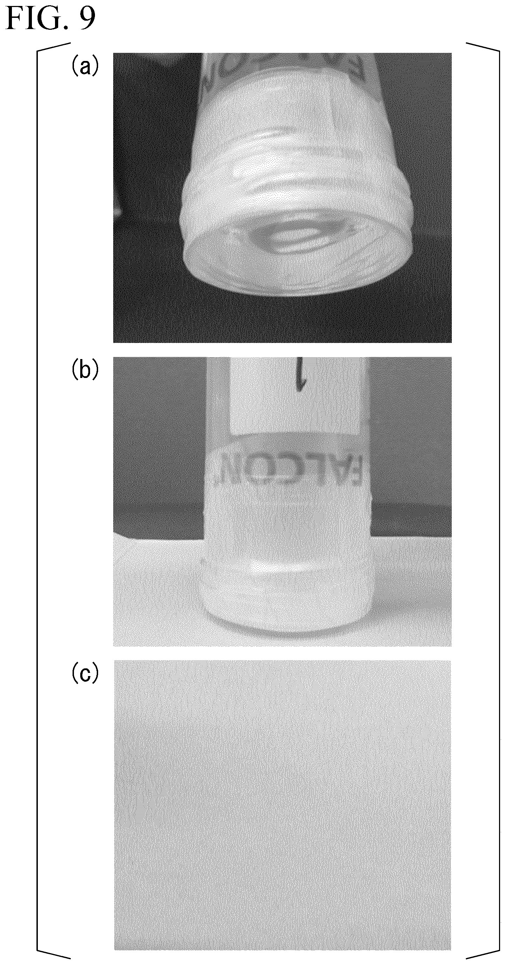

Fig. 9A is a photograph showing a state in which a conical tube to which a dried product of an atelocollagen vitrigel membrane having no through-holes was fixed was inverted. Fig. 9B is a photograph showing a state in which the inverted conical tube was brought into contact with cardboard. Fig. 9C is a photograph of a state in which no trace of water was observed as a result of contact with the cardboard. As a result, it became clear that water did not permeate at all.

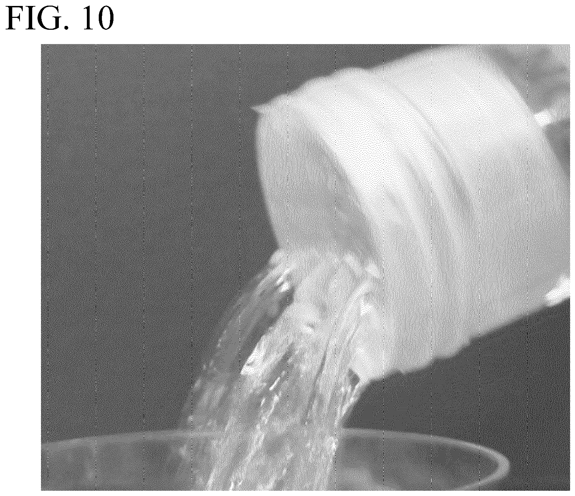

Fig. 10 is a photograph showing a state in which a conical tube to which the dried product of the hydrogel membrane of Production Example 2 was fixed was inverted. As a result, it was observed that water permeated vigorously.

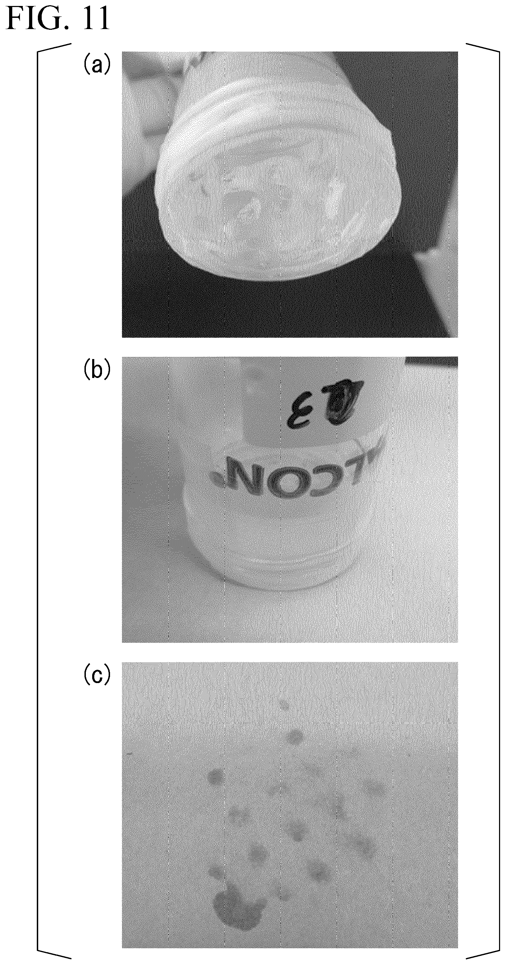

Fig. 11A is a photograph showing a state in which a conical tube to which the dried product of the hydrogel membrane of Production Example 1 was fixed was inverted. Fig. 11B is a photograph showing a state in which the inverted conical tube was brought into contact with the cardboard about 15 minutes after the conical tube was inverted. Fig. 11C is a photograph of a state in which traces of water were observed as a result of contacting the cardboard. As a result, it was clarified that the hydrogel membrane of Production Example 1 has liquid permeability to the extent that the liquid gradually exudes.

### [Industrial Applicability]

According to the present invention, it is possible to provide a technique for adhering a hydrogel membrane to an uneven surface.

### [Reference Signs List]

100 Hydrogel film
110 One surface
120 The other surface
130 Through-hole
200 Production apparatus
210 Bottom surface portion
220 Side surface portion
230 Container portion
240 Columnar jig

## Claims

1. A hydrogel membrane comprising a plurality of through-holes penetrating one surface and the other surface.

2. The hydrogel membrane according to Claim 1, wherein an opening area of the through-holes on the one surface is different from an opening area on the other surface.

3. The hydrogel membrane according to Claim 1 or 2, wherein the through-holes have a diameter of 0.1 µm to 5 mm.

4. The hydrogel membrane according to Claim 1 or 2, wherein the through-holes have a number density of 1 to 1,000,000/cm².

5. The hydrogel membrane according to any one of Claims 1 to 4, wherein the hydrogel contains native collagen or atelocollagen.

6. A dried product of the hydrogel membrane according to any one of Claims 1 to 5.

7. An apparatus for producing the hydrogel membrane according to any one of Claims 1 to 5 or the dried product of the hydrogel membrane according to Claim 6, the apparatus comprising:
a container portion having a bottom surface portion and a side surface portion; and
a plurality of columnar jigs configured to be arranged in contact with the bottom surface portion.

8. A method for producing a hydrogel having a plurality of through-holes penetrating one surface and the other surface, the method comprising:
(a) injecting a sol into a container portion of a production apparatus including the container portion having a bottom surface portion and a side surface portion and a plurality of columnar jigs configured to be arranged in contact with the bottom surface portion;
(b) arranging the plurality of columnar jigs to be in contact with the bottom surface portion before or after injecting the sol;
(c) allowing the sol to stand and gelate to obtain a hydrogel; and
(d) removing the plurality of columnar jigs to obtain a hydrogel having a plurality of through-holes penetrating one surface and the other surface.

9. A method for producing a dried product of a hydrogel having a plurality of through-holes penetrating one surface and the other surface, the method comprising:
(e) drying the hydrogel obtained by the production method according to Claim 8 to obtain a dried product of the hydrogel.

10. A method for producing a dried product of a hydrogel membrane having a plurality of through-holes penetrating one surface and the other surface, the method comprising:
(f) hydrating the dried product of the hydrogel obtained by the production method according to Claim 9 to obtain a hydrated hydrogel membrane.

11. A method for producing a dried product of a hydrogel membrane having a plurality of through-holes penetrating one surface and the other surface, the method comprising:
(e2) drying the hydrogel membrane obtained by the production method according to Claim 10 to obtain a dried product of the hydrogel membrane.
